# EUROPEAN PATENT SPECIFICATION

(11) **EP 4 374 865 B1**
(45) Date of publication and mention of the grant of the patent: **07.10.2026**
(21) Application number: 22846010.1
(22) Date of filing: 28.04.2022
(51) Int. Cl.: A61K 36/232, A61K 36/8984, A61K 36/53, A61K 35/32, A61K 36/234, A61K 36/481, A61K 36/21, A61P 19/08, A61K 36/28, A61K 36/35, A61K 36/704

(54) **POWDER, PILL, OR LIQUID FORM HERBAL MEDICINE COMPOSITION FOR FRACTURE RESTORATION AND BONE REGENERATION SPEED IMPROVEMENT, AND POWDER, PILL, OR LIQUID FORM HERBAL MEDICINE PREPARATION USING SAME**
PULVER-, PILLEN- ODER FLÜSSIGFORM-KRÄUTERMEDIZINZUSAMMENSETZUNG ZUR WIEDERHERSTELLUNG VON FRAKTUREN UND VERBESSERUNG DER KNOCHENREGENERATIONSGESCHWINDIGKEIT SOWIE PULVER-, PILLEN- ODER FLÜSSIGFORM-KRÄUTERMEDIZINPRÄPARAT DAMIT
COMPOSITION À BASE DE PLANTES MÉDICINALES SOUS FORME DE POUDRE, DE PILULE OU DE LIQUIDE POUR LA RESTAURATION DES FRACTURES ET L'AMÉLIORATION DE LA VITESSE DE RÉGÉNÉRATION OSSEUSE, ET PRÉPARATION À BASE DE PLANTES MÉDICINALES SOUS FORME DE POUDRE, DE PILULE OU DE LIQUIDE UTILISANT CELLE-CI

(30) Priority: 22.07.2021 KR 20210096593
(43) Date of publication of application: 29.05.2024
(73) Proprietor: Choi, Young Jin, Seoul 05555 (KR)
(72) Inventor: Choi, Young Jin, Seoul 05555 (KR)
(74) Representative: Plougmann Vingtoft a/s
(86) International application number: PCT/KR2022/006073
(87) International publication number: WO 2023/003138

(56) References cited:
- CN-A- 113 082 151
- KR-A- 20190 135 652
- KR-A- 20200 033 241
- KR-B1- 100 731 160
- KR-B1- 102 164 421
- KR-B1- 102 355 277

## Description

### Technical Field

The present invention relates to an herbal composition in powder, pill or liquid form for speeding up fracture healing and bone regeneration and an herbal formulation in powder, pill or liquid form containing the same, and more specifically, to an herbal composition in powder, pill or liquid form for speeding up fracture healing and bone regeneration, which may promote fracture healing and bone regeneration by containing medicinal herbs capable of improving fracture healing and bone regeneration and medicinal herbs capable of enhancing digestion and absorption, and an herbal formulation in powder, pill or liquid form containing the same.

### Background Art

As used herein, the term "fracture" refers to a phenomenon in which bones in a part of the body are destroyed. This fracture can be healed when two separate bones are joined by the formation of new tissue around the fracture due to the influences of various physical and biological factors.

In recent years, various methods for promoting fracture healing have been proposed, and examples thereof include a method of slowing venous blood flow, a method of stimulating sympathetic nerves, a method of applying electrical stimulation, a method of administering hormones, or methods of administering specific compounds, for example, vitamin D, vitamin D derivatives, BMP, and the like.

However, a satisfactory fracture healing method or fracture healing agent has not yet been developed.

Meanwhile, a healing method for bone fracture caused by a physical external force comprises performing a surgical operation and then fixing the bone with a splint or a plaster bandage so that the bone fracture is naturally healed. However, this healing method has a problem in that the fracture healing period is long.

To overcome this problem, the present applicant disclosed, in Korean Patent No. 10-0731160, an herbal composition for promoting fracture healing containing 18 to 23 wt% of Angelica gigas, 18 to 23 wt% of Cnidium officinale, 7.5 to 9.5 wt% of Astragalus membranaceus, 7.5 to 9.5 wt% of ginseng, 7.5 to 9.5 wt% of Lyciumchinense, 7.5 to 9.5 wt% of Codonopsispilosula, 3.5 to 4.5 wt% of Cuscutachinensis, 3.5 to 4.5 wt% of Dipsaci radix, 3.5 to 4.5 wt% of Dendrobium moniliforme, 3.5 to 4.5 wt% of Psoraleacorylifolia, 3.5 to 4.5 wt% of Albizziae cortex, and 3.5 to 4.5 wt% of velvet antler.

However, although the herbal composition has an effect of promoting fracture healing, but it contains only medicinal herbs known to be effective in fracture healing, and thus has the problem of causing indigestion, digestive disorder, etc.

### DISCLOSURE

### Technical Problem

The present invention has been made in order to solve the above-described problems, and an object of the present invention is to provide an herbal composition in powder, pill or liquid form for speeding up fracture healing and bone regeneration, which contains specific medicinal herbs known to be effective in bone healing and bone regeneration as well as medicinal herbs capable of enhancing digestion, and thus has an enhanced ability to be digested and absorbed and may promote the healing of fracture sites while allowing the medicinal herbal ingredients to be absorbed quickly in the body when taken, and an herbal formulation in powder, pill or liquid form containing the herbal composition.

### Technical Solution

To achieve the above object, the present invention provides an herbal composition in powder, pill or liquid form for speeding up fracture healing and bone regeneration containing 20 parts by weight of Angelica gigas, 16 parts by weight of Dendrobium moniliforme, 12 parts by weight of Dipsaci radix, 10 parts by weight of antler, 8 parts by weight of Cnidium officinale, 8 parts by weight of Astragalus membranaceus, and 4 parts by weight of Achyranthes japonica.

The present invention also provides an herbal composition in powder, pill or liquid form for speeding up fracture healing and bone regeneration containing 20 parts by weight of Angelica gigas, 16 parts by weight of Dendrobium moniliforme, 12 parts by weight of Dipsaci radix, 10 parts by weight of antler, 8 parts by weight of Cnidium officinale, 8 parts by weight of Astragalus membranaceus, 4 parts by weight of Achyranthes japonica, and 2 parts by weight of Massa medicata fermentata.

The present invention also provides an herbal composition in powder, pill or liquid form for speeding up fracture healing and bone regeneration containing 10 parts by weight of Angelica gigas, 8 parts by weight of Dendrobium moniliforme, 6 parts by weight of Dipsaci radix, 5 parts by weight of antler, 4 parts by weight of Cnidium officinale, 4 parts by weight of Astragalus membranaceus, 2 parts by weight of Achyranthes japonica, and 4 parts by weight of Massa medicata fermentata.

The present invention also provides an herbal composition in powder, pill or liquid form for speeding up fracture healing and bone regeneration containing 20 parts by weight of Angelica gigas, 16 parts by weight of Dendrobium moniliforme, 12 parts by weight of Dipsaci radix, 10 parts by weight of antler, 8 parts by weight of Cnidium officinale, 8 parts by weight of Astragalus membranaceus, 4 parts by weight of Achyranthes japonica, and 4 parts by weight of Massa medicata fermentata.

### Advantageous Effects

The effects of the present invention are as follows. Since the herbal composition of the present invention contains Angelica gigas, Dendrobium moniliforme, Dipsaci radix, antler, Cnidium officinale, Astragalus membranaceus, and Achyranthes japonica, which are effective in fracture healing and bone regeneration, as well as Massa medicata fermentata capable of enhancing digestion, it has an enhanced ability to be digested and absorbed, and when a patient in need of fracture healing takes the herbal composition, the medicinal herbal ingredients may be absorbed quickly into the body and promote healing of the fracture site, thereby greatly shortening the fracture healing period. In addition, since the composition of the present invention is prepared in powder, pill or liquid form, it may be easily and conveniently taken by the patient.

### Description of the Drawings

FIG. 1 is a graph showing post-surgical bone gap changes for a control group to which the liquid herbal composition of the present invention was not administered, and groups (Comparative Example 1 and Examples 1 to 4) to which the herbal composition was administered.
FIG. 2 shows X-ray photographs used to calculate the speed of bone regeneration after administration of the herbal composition of the present invention following fracture surgery.
FIGS. 3, 4, 5, 6, 7, and 8 are X-ray photographs of the control group after fracture surgery, and X-ray photographs of the herbal composition-administered group before and after administration of the herbal composition after fracture surgery.
FIGS. 9, 10, 11, 12, 13, and 14 are representative histological photographs of peri-fracture tissues in the control group and the herbal composition-administered groups.
FIG. 15 is a graph showing the length of the callus formed at the fracture line in each of the control group and the herbal composition-administered groups.
FIG. 16 is a graph comparing the effects of herbal composition E according to Example 4 and herbal composition A according to Comparative Example 1.

### Mode for Invention

An herbal composition in powder, pill or liquid form for speeding up fracture healing and bone regeneration according to one embodiment of the present invention contains 20 parts by weight of Angelica gigas, 16 parts by weight of Dendrobium moniliforme, 12 parts by weight of Dipsaci radix, 10 parts by weight of antler, 8 parts by weight of Cnidium officinale, 8 parts by weight of Astragalus membranaceus, and 4 parts by weight of Achyranthes japonica.

An herbal composition in powder, pill or liquid form for speeding up fracture healing and bone regeneration according to another embodiment of the present invention contains 20 parts by weight of Angelica gigas, 16 parts by weight of Dendrobium moniliforme, 12 parts by weight of Dipsaci radix, 10 parts by weight of antler, 8 parts by weight of Cnidium officinale, 8 parts by weight of Astragalus membranaceus, 4 parts by weight of Achyranthes japonica, and 2 parts by weight of Massa medicata fermentata.

An herbal composition in powder, pill or liquid form for speeding up fracture healing and bone regeneration according to still another embodiment of the present invention contains 10 parts by weight of Angelica gigas, 8 parts by weight of Dendrobium moniliforme, 6 parts by weight of Dipsaci radix, 5 parts by weight of antler, 4 parts by weight of Cnidium officinale, 4 parts by weight of Astragalus membranaceus, 2 parts by weight of Achyranthes japonica, and 4 parts by weight of Massa medicata fermentata.

An herbal composition in powder, pill or liquid form for speeding up fracture healing and bone regeneration according to yet another embodiment of the present invention contains 20 parts by weight of Angelica gigas, 16 parts by weight of Dendrobium moniliforme, 12 parts by weight of Dipsaci radix, 10 parts by weight of antler, 8 parts by weight of Cnidium officinale, 8 parts by weight of Astragalus membranaceus, 4 parts by weight of Achyranthes japonica, and 4 parts by weight of Massa medicata fermentata.

The present invention also provides an herbal formulation which contains, as an active ingredient, the herbal composition in powder, pill or liquid form for speeding up fracture healing and bone regeneration according to the present invention, and is prepared in powder, pill or liquid form.

Hereinafter, preferred embodiments of the present invention will be described in detail.

The Angelica gigas of the present invention, which can speed up fracture and bone regeneration, has the effects of nourishing the blood, relieving pain by activating blood circulation, and increasing intestinal energy absorption, and is used for symptoms, such as syndrome of heart-liver blood deficiency, menstrual pain caused by abnormal menstrual cycle, menopause, afterpain, contusion injury, pain caused by a large abscess, and constipation. The effect of Angelica gigas on the proliferation of human bone cells was already reported (Clinica Chimica Acta 324 (2002) 89-97, Effect of Angelica sinensis on the proliferation of human bone cells).

As Angelica gigas having such effects, the Angelica gigas root collected in the fall is used in a dry state. The content of Angelica gigas is preferably 15 to 30 wt% based on the total weight of the herbal composition. If the content of Angelica gigas in the herbal composition is excessively high, problems of edema and diarrhea may arise.

Dendrobium moniliforme that is used in the present invention is an evergreen perennial grass belonging to the family Orchidaceae, is about 20 cm in height, and lives attached to rocks or old trees, and a lot of thick roots sprout from the rhizome. Several stems come out and grow straight, and the old ones have no leaves on the stems and have only nodes. The leaves are 2 to 3 years old, come out alternately, and are 4 to 7 cm in length and about 1 cm in width. Dendrobium moniliforme has the effects of promoting the secretion of gastric juice by strengthening the stomach, and removing fever by cultivating yin energy, and is used to treat symptoms such as thirst caused by fever, fever caused by weak yin, and lack of strength in the back and knees. In addition, Dendrobium moniliforme acts selectively on the Kidney meridian to strength the kidney and bone.

If the content of Dendrobium moniliforme in the herbal composition is excessively high, a problem of fever may arise. Thus, the content of Dendrobium moniliforme is preferably 15 to 20 wt% based on the total weight.

The term "Dipsaci radix" refers to the root of Dipsacus asperoides C Y. Cheng et T. M. Ai which is a perennial herb belonging to the family Dipsacaceae. Dipsaci radix is a kind of food and herbal medicine that has been used by people since ancient times. In recent years, as the effects of Dipsaci radix on growth hormone secretion and growth promotion have been revealed, the demand has gradually expanded, and research on the safety and efficacy thereof has been actively conducted.

Dipsaci radix has the effects of strengthening the liver and kidney and connecting broken bones or muscles, and is used to treat symptoms such as the lack of proper blood of the liver and kidney, back and leg pain, bleeding from the uterus during pregnancy, and a disease in which muscles (tendons) are damaged and bones are broken.

The known pharmacological actions of Dipsaci radix include antidiabetic effects, muscle and joint pain alleviation action, anticomplement effects, treatment of cough asthma caused by decreased physical strength, and treatment of diarrhea. Continuous research on the new efficacy of Dipsaci radix extracts is in progress in various fields.

As Dipsaci radix in the present invention, one obtained by harvesting and drying the roots of Dipsacus asperoides C Y. Cheng et T. M. Ai and removing the hair and beard from the roots is used. The content of Dipsaci radix is 10 to 15 wt% based on the total weight of the herbal composition. If the content of Dipsaci radix is excessively high, a problem of fever may arise.

The antler that is used in the present invention is obtained by cutting and drying the ossified horns of Cervus nippon Temminck, Cervus elaphus Linne or Cervus canadensis Erxleben belonging to the family Cervidae. The antler has the effects of nourishing the vitality of the kidneys, boosting yang energy, promoting bone growth by promoting the synthesis of proteins and nucleic acids, promoting growth, promoting hematopoiesis, and strengthening the heart, and is known to improve immune function and to be effective against liver disease and osteoporosis.

If the content of the antler is excessively high, a problem of diarrhea may arise. Thus, the content of the antler is preferably 10 to 15 wt% based on the total weight of the herbal composition.

Cnidium o*ff*icinale that is used in the present invention is a perennial dicotyledonous plant belonging to the family Umbelliferae, and is native to China. It has the effects of facilitating blood circulation, helping the operation of Qi smoothly, removing gout, and stopping pain, and is used to treat symptoms such as menstrual irregularities, menopause menstrual pain, pain after difficult delivery, quadriplegia, contusion injury, and headache.

As Cnidium o*ff*icinale having such effects, one obtained by collecting the root in September through November, removing the small branches and then drying the root is used. The content of Cnidium o*ff*icinale is preferably 5 to 10 wt% based on the total weight of the herbal composition. If the content of Cnidium o*ff*icinale in the herbal composition is excessively high, a problem of fever may arise. It is considered that, although Cnidium o*ff*icinale does not exert a direct effect on fractures, it further promotes fracture healing by exhibiting the effect of treating inflammation by the action of circulating blood circulation.

Astragalus membranaceus that is used in the present invention is a medicinal herb that protects the functions of the skin, prevents sweating, gives new flesh, and helps the immune function.

Astragalus membranaceus has the effects of nourishing qi, boosting yang qi, protecting and firming the skin, removing toxins such as inflammation, giving new flesh, facilitating moisture drainage, and removing edema, and is used to treat symptoms such as spleen Gi deficiency, symptom of sunken middle qi, symptoms of weakness in both the nasal and lungs, a condition in which the body is weak and has no energy, excessive sweating, a big boil, weakness or lack of energy, and hydropsy.

Astragalus membranaceus is a good medicinal herb for obese people, because it controls the amount of sweat and is used as a treatment for diabetes due to its diuretic action.

Whole body sweating, dizziness and exhaustion are evidence that the qi has become weak. In addition to these symptoms, Astragalus membranaceus is also effective for boredom and lethargy. Astragalus membranaceus has the effects of suppressing sweating, strengthening the skin, draining pus, alleviating swelling, and giving new flesh. In addition, it relieves chronic fatigue and is effective in improving insomnia and weak constitution. It contains alkaloids, amino acids, aspartic acid, and the like. It is sweet in taste and slightly warm in nature. It acts on nasal meridian, lung meridian, triple energizer meridian, and nerve. Astragalus membranaceus strengthens Qi and acts as a tonic and cardiac tonic.

Astragalus membranaceus helps blood circulation due to its vasodilation action, and increases the resistance of capillaries. As Astragalus membranaceus in the present invention, the 2-5 year-old root collected in the fall or spring is used after drying of the peel. In the present invention, Astragalus membranaceus known to have the above-described effects is added in order to allow a bone fracture in the human body to be healed more quickly through the effect of strengthening energy in fracture healing.

As Astragalus membranaceus, the rood collected in spring and autumn is used after drying and removal of the beard and hair from the root. If the content of Astragalus membranaceus in the herbal composition is excessively high, a problem of indigestion or fever may arise. Thus, the content of Astragalus membranaceus is preferably 5 to 10 wt% based on the total weight of the herbal composition.

Achyranthes japonica that is used in the present invention has a node having a shape similar to that of a cow's knee in the plant stem. Achyranthes japonica is a medicinal herb corresponding to the Achyranthes root, and contains saponin and a large amount of calcium as active ingredients, and is known to have a remarkable effect on the treatment of knee diseases (arthritis, rheumatoid arthritis, and inflammation caused by bruises). It is also frequently used in the case in which the waist and legs feel heavy and are painful and sometimes when there are muscle spasms. In addition, Achyranthes japonica is also used to relieve cerebral blood vessel spasms while lowering blood pressure when there are symptoms such as headache, dizziness, and eye irritation in hypertension. Young shoots thereof are eaten as herbs, and the root is also used for neuralgia in folk remedy. If the content of Achyranthes japonica in the herbal composition is excessively high, it may cause diarrhea. Thus, the content of Achyranthes japonica is preferably 2 to 5 wt% based on the total weight of the herbal composition.

As used herein, " Massamedicata*f*ermentata" in oriental medicine refers to a medicinal herb made by mixing wheat flour, bran, cocklebur juice, Glycine soja juice, Sweet annie juice, Apricot kernel extract, red bean paste, etc. and fermenting the mixture together with yeast. It is known that Massamedicata*f*ermentata mainly acts on the spleen and stomach to strengthen the function of the digestive system, has an action to comfort the stomach with the action to help digestion, and exhibits effects against an upset stomach, a stuffy chest, vomiting and diarrhea, and abdominal pain due to extravasated blood after childbirth

Massamedicata*f*ermentata serves to enhance the digestion of the herbal composition by the above-mentioned efficacy, thereby improving the absorption of the herbal composition when taken, so that the effects of the medicinal herbs can be quickly obtained. If the content of Massamedicata*f*ermentata is excessively low, it may be difficult to obtain the efficacy of Massa medicata fermentata. If the content of Massa medicata fermentata is excessively high, it may cause drowsiness. Thus, the content of Massamedicata*f*ermentata is preferably 2 to 5 wt% based on the total weight of the herbal composition.

### Comparative Example 1 (Liquid Herbal Composition A: Korean Patent Application No. 10-0731160 Filed in the Name of this Applicant)

Medicinal herbs in an amount corresponding to 10 packs, each consisting of 20 g of Angelica gigas, 20 g of Cnidium officinale, 8 g of ginseng, 8 g of Lyciumchinense, 8 g of Codonopsispilosula, 4 g of Dipsaci radix, 4 g of Dendrobium moniliforme, 4 g of Psoraleacorylifolia, 4 g of Cuscutachinensis, 4 g of Albizziae cortex, and 4 g of antler, were added to 6,000 ml of water and decocted in a pot at 95 to 100° C for 2 to 4 hours, and the medicinal herbs were filtered out to obtain 1,800 ml of a final liquid herbal composition.

### Example 1 (Liquid Herbal Composition B: Base)

Medicinal herbs in an amount corresponding to 10 packs, each consisting of 20 g of Angelica gigas, 16 g of Dendrobium moniliforme, 12 g of Dipsaci radix, 10 g of antler, 8 g of Cnidium officinale, 8 g of Astragalus membranaceus, and 4 g of Achyranthes japonica, were added to 6,000 ml of water and decocted in a pot at 95 to 100° C for 2 to 4 hours, and the medicinal herbs were filtered out to obtain 1,200 ml of a final liquid herbal composition.

### Example 2 (Liquid Herbal Composition C: Liquid Herbal Composition B + 2 g of Massa medicata fermentata)

A liquid herbal composition having a final volume of 1,200 ml was obtained in the same manner as in Example 2, except that 2 g of Massa medicata fermentata was added.

### Example 3 (Liquid Herbal Composition D: ½ Liquid Herbal Composition B + 4 g of Massa medicata fermentata)

Medicinal herbs in an amount corresponding to 10 packs, each consisting of 10 g of Angelica gigas, 8 g of Dendrobium moniliforme, 6 g of Dipsaci radix, 5 g of antler, 4 g of Cnidium officinale, 4 g of Astragalus membranaceus, 2 g of Achyranthes japonica, and 4 g of Massamedicata*f*ermentata, were added to 6,000 ml of water and decocted in a pot at 95 to 100° C for 2 to 4 hours, and the medicinal herbs were filtered out to obtain 1,200 ml of a final liquid herbal composition.

### Example 4 (Herbal Composition E: Herbal Composition B + 4 g of Massa Medicata fermentata

Medicinal herbs in an amount corresponding to 10 packs, each consisting of 20 g of Angelica gigas, 16 g of Dendrobium moniliforme, 12 g of Dipsaci radix, 10 g of antler, 8 g of Cnidium officinale, 8 g of Astragalus membranaceus, 4 g of Achyranthes japonica, and 4 g of Massamedicata*f*ermentata, were added to 6,000 ml of water and decocted in a pot at 95 to 100° C for 2 to 4 hours, and the medicinal herbs were filtered out to obtain 1,200 ml of a final liquid herbal composition.

As in Examples 1 to 4, the herbal composition of the present invention is preferably prepared in liquid form and taken as a liquid, but is not limited thereto and may also be prepared in powder or pill form. The herbal composition in pill form may be prepared by preparing the herbal composition in liquid form, extracting the herbal composition with hot water or ethanol, concentrating and drying the extract, and forming the dried extract into a pill. The herbal composition in powder form may be prepared by powdering the pill. However, the method of preparing the herbal composition of the present invention in liquid, powder or pill form is not critical to the present invention, and detailed description thereof will be omitted herein.

### Experimental Example 1

In order to examine whether liquid herbal formulations A, B, C, D and E obtained in Comparative Example 1 and Examples 1 to 4 above would speed up bone fracture and bone regeneration, an experiment was performed under the following conditions.

### 1. Experimental Method

In order to examine the effect of each of the liquid herbal compositions on bone healing, water and each of the liquid herbal compositions were administered to 7-week-old SD rats with fractures, and then X-ray imaging was performed.

The administration frequency and dose of each test substance for each group are as follows.
Group control: vehicle control (water)
Group A: liquid herbal composition A (10 ml/kg p.o)
Group B: liquid herbal composition B (10 ml/kg p.o)
Group C: liquid herbal composition C (10 ml/kg p.o)
Group D: liquid herbal composition D (10 ml/kg p.o)
Group E: liquid herbal composition E (10 ml/kg p.o)

### 2. Measurement

### 1) Measurement of Bone Regeneration Rate

Water and each of liquid herbal compositions A, B, C, D and E were administered orally every day to the experimental animals of each group, and to examine the bone regeneration speed, X-ray imaging was performed weekly to measure the ulnar gap.

### 2) Measurement of Callus Formation Rate

After oral administration of liquid herbal composition A, B, C, D or E for 4 weeks, 2 mm portions with respect to the ulnar fracture line were extracted and subjected to H & E staining. The length of the callus regenerated from the fracture line was measured by observing the stained area with an Olympus microscope.

### 3. Experimental Results

### 1) Effect of Liquid Herbal Composition A, B, C, D or E on Bone Regeneration

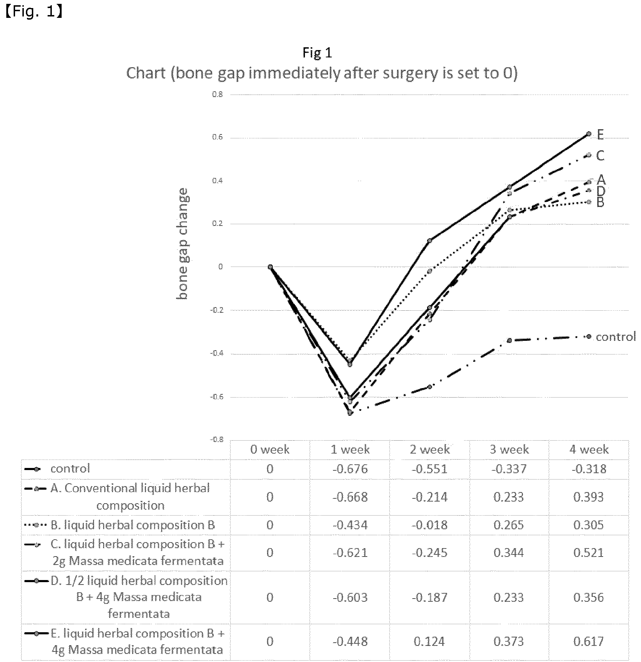

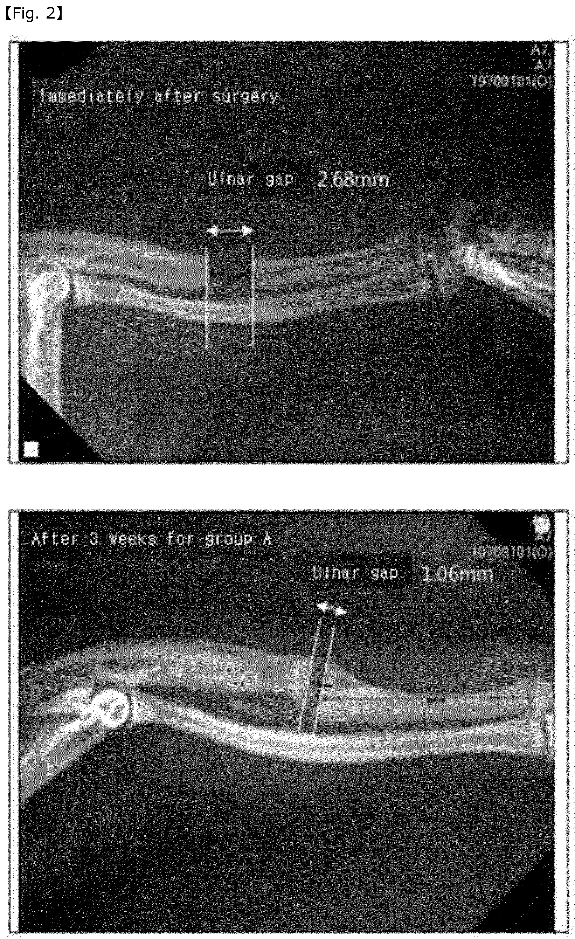

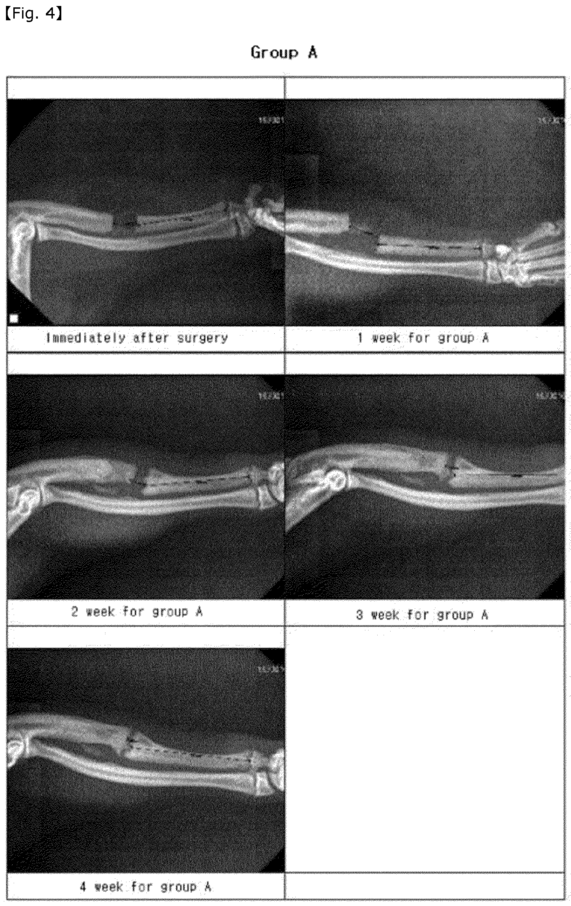

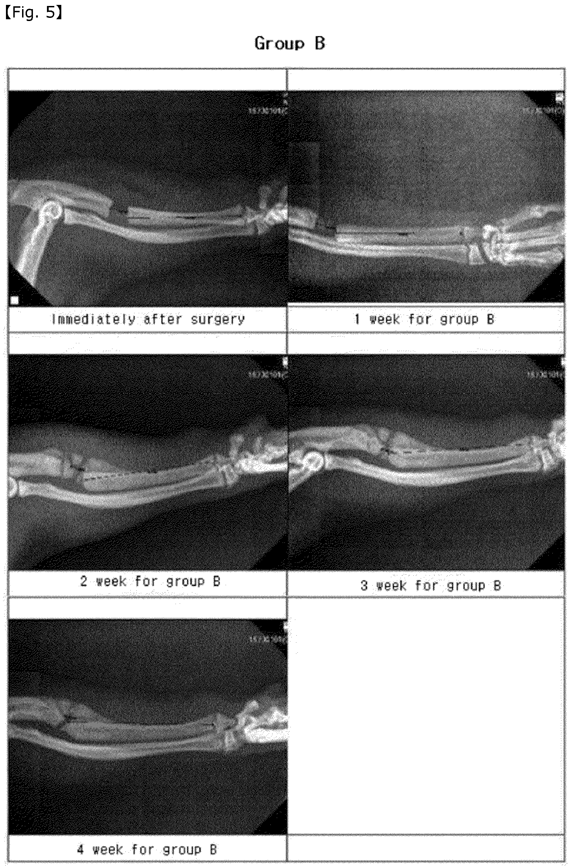

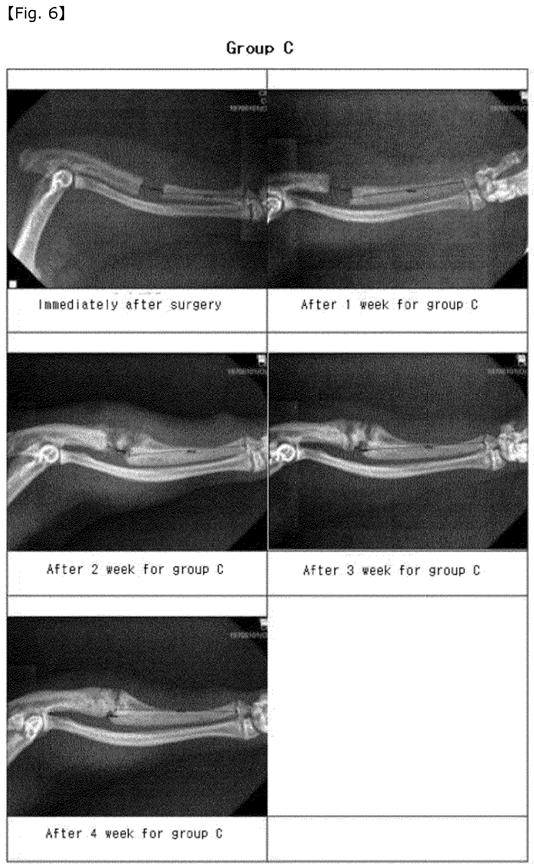

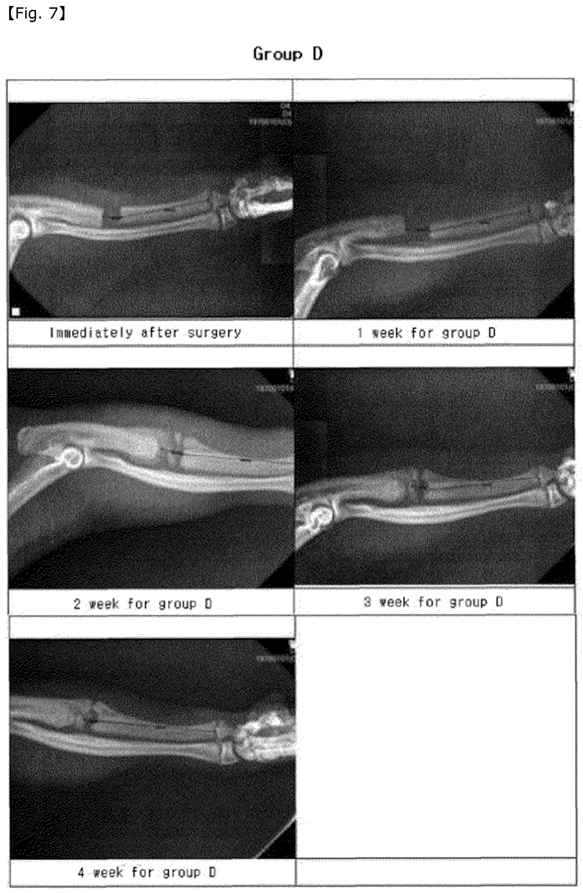

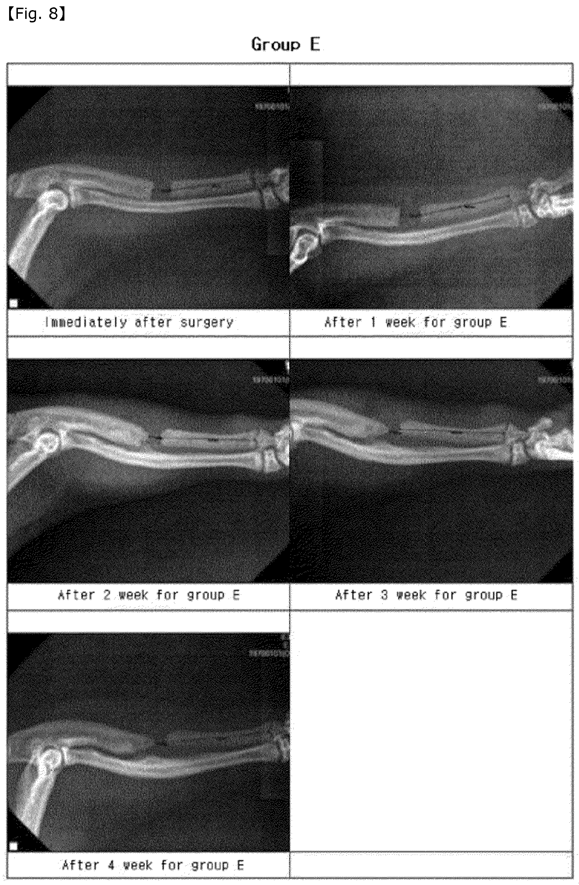

After fracture induction, liquid herbal composition A, B, C, D or E was administered p.o. daily at a dose of 10 ml/kg p.o, and X-ray imaging was performed to measure the ulnar gap. The results are shown in FIGS. 1 and 3 to 8. (Method for calculating bone regeneration rate: as shown in FIG. 2, ulnar gap immediately after surgery (2.68 mm) - ulnar gap after 3 weeks (1.06 mm)=1.62 mm. As the callus is formed and bone is regenerated, the value increases.)

As shown in FIG. 1, until 1 week after administration during the experiment, no difference was seen between the groups, but after 2 weeks, the group to which liquid herbal composition E was administered started to show a significant difference from the control group, and after 3 weeks, the groups to which liquid herbal compositions A, B, C, D and E were administered, respectively, all showed significant differences from the control group.

Looking at the experimental results depending on time, the control group showed a bone regeneration rate of -15% at weeks 3 and 4, but the group to which liquid herbal composition E was administered showed increases in bone regeneration rates of 4.7% at week 2, 14.3% at week 3, and 23.6% at week 4. The group to which liquid herbal composition C was administered showed increases in bone regeneration rates of 13.4% at week 3 and 20.5% at week 4; the group to which liquid herbal composition A was administered showed increases in bone regeneration rates of 9.6% at week 3 and 16.3% at week 4; the group to which liquid herbal composition D was administered showed increases in bone regeneration rates of 10.3% at week 3 and 15.7% at week 4; and the group to which liquid herbal composition B was administered showed increases in bone regeneration rates of 10.6% at week 3 and 12.3% at week 4.

### 2) Results of X-Ray Imaging Before and After Administration of Liquid Herbal Composition

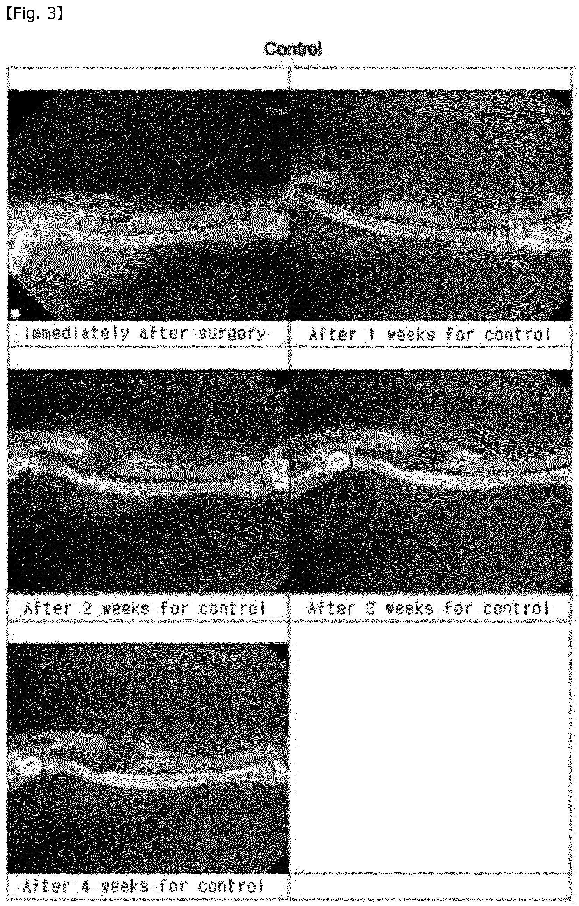

FIGS. 3 to 8 show representative X-ray photographs taken every week. As shown therein, it can be seen that bone regeneration was more accelerated in the liquid herbal composition-administered group than in the control group.

### 3) Representative Histological Photographs of Peri-Fracture Tissues and Length of Callus Formed at Fracture Line

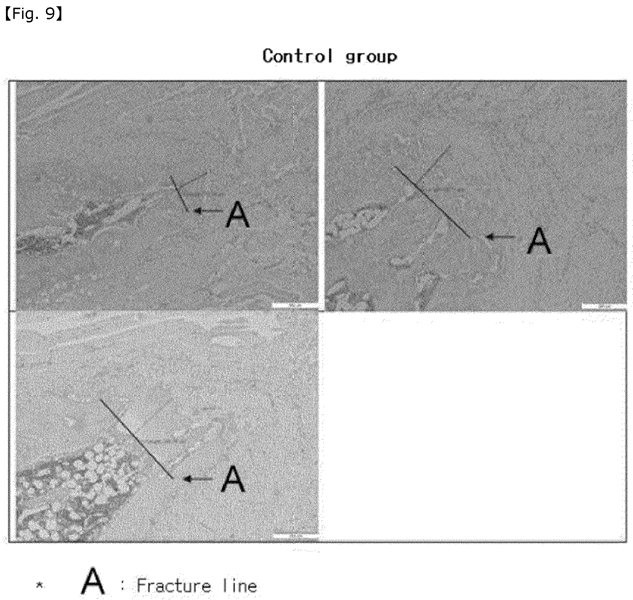

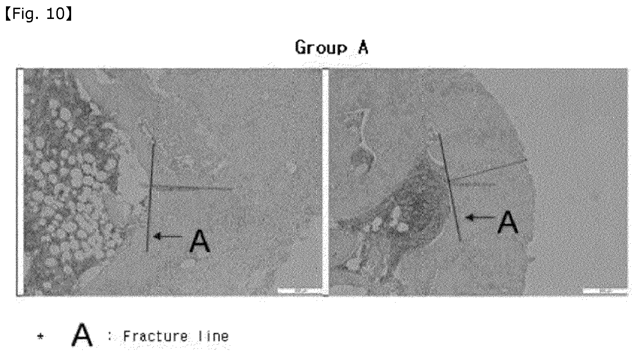

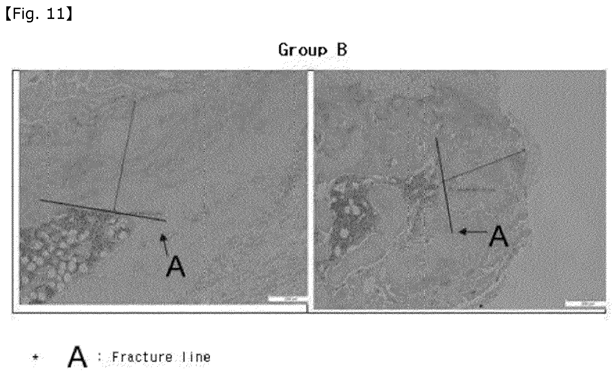

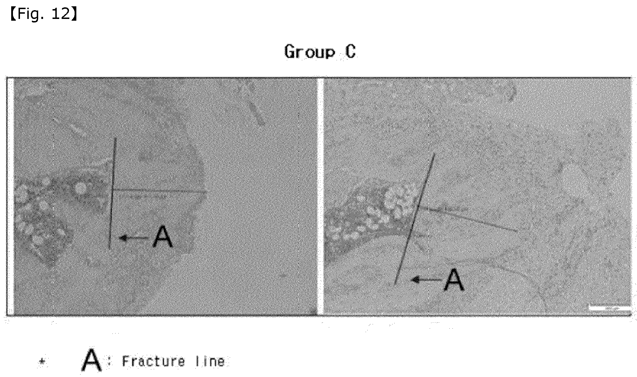

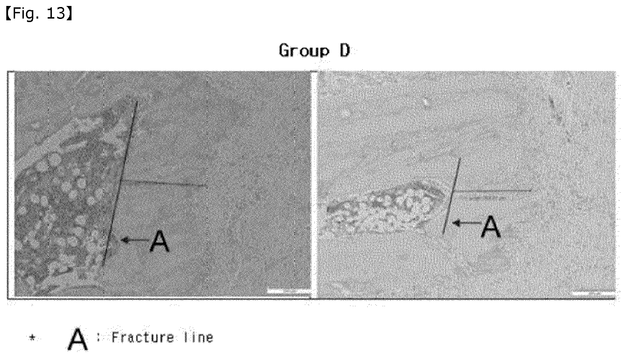

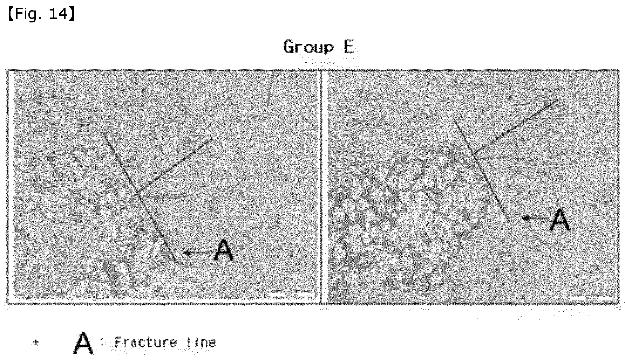

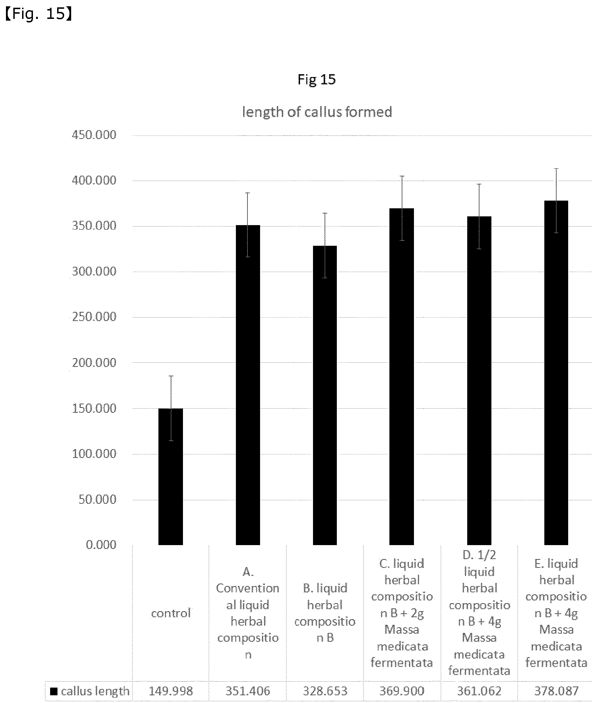

FIGS. 9 to 14 show representative histological photographs of peri-fracture tissues, and FIG. 15 is a graph showing the length of the callus formed at the fracture line. As shown therein, it can be seen that the bone (callus) regenerated from the fracture line was formed more in the liquid herbal composition-administered groups than in the control group.

### 4) Comparison of Effects of Liquid Herbal Composition E and Conventional Liquid Herbal Composition (Comparative Example 1)

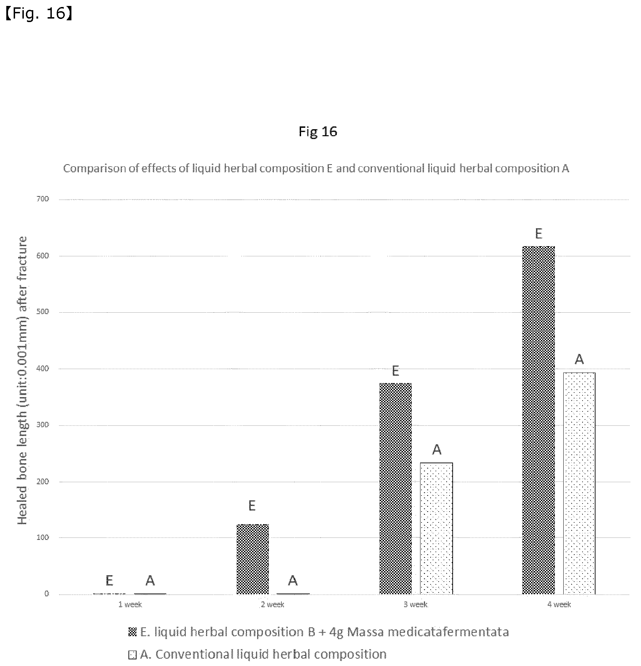

As a result of comparing the effect of liquid herbal composition E of Example 4 with the effect of conventional liquid herbal composition A of Comparative Example 1, as shown in the graph in FIG. 16, it can be seen that, in the case of the group to which liquid herbal composition E was administered, bone healing started from 2 weeks after fracture occurrence, and 4 weeks, the bone healing effect of liquid herbal composition E increased by 57% compared to that of liquid herbal composition A.

### 4. Conclusions

This experiment was performed to examine the effects of liquid herbal compositions A, B, C, D and E on fracture healing and bone regeneration. In this experiment, each liquid herbal composition was administered orally to the experimental animals with fracture, and then X-ray imaging was performed to measure the ulnar gap.

As a result, it could be confirmed that, until 1 week after administration, there was no difference in fracture gap between the groups, but after 2 weeks, the group to which liquid herbal composition E was administered showed a significant difference from the control group, and after 3 weeks, the groups to which liquid herbal compositions A, B, C, D and E, respectively, all showed increased bone regeneration rates compared to the control group.

In particular, it was confirmed that the bone regeneration rates at week 4 decreased by -15% in the control group, but increased by 23.6% in the liquid herbal composition E-administered group, 20.5% in the liquid herbal composition C-administered group, 16.3% in the liquid herbal composition A-administered group, 15.7% in the liquid herbal composition D-administered group, and 12.3% in the liquid herbal composition B-administered group.

From the results of X-ray imaging, it could be seen that bone regeneration was faster in the groups to which liquid herbal compositions A, B, C, D and E, respectively, than in the control group. In addition, from the histological photographs, it could be confirmed that the callus (regenerated bone) was more formed in the groups to which liquid herbal compositions A, B, C, D and E, respectively, than in the control group.

In particular, it could be confirmed that, in the case of the group to which liquid herbal composition E was administered, bone healing started from 2 weeks after fracture occurrence, and 4 weeks, the bone healing effect of liquid herbal composition E increased by 57% compared to that of liquid herbal composition A. These results show that, in addition to Angelica gigas, Dendrobium moniliforme, Dipsaci radix, antler, Cnidium o*ff*icinale and Astragalus membranaceus, Achyranthes japonica known to be effective in arthritis treatment and Massa medicata fermentata that enhances the digestion and absorption of the herbal ingredients of the liquid herbal composition serve to greatly speed up bone healing and bone regeneration.

In addition, during the first week after inducing a fracture, the bone gap becomes wider due to the activity of osteoclasts. Looking at the values at week 1 in FIG. 1, the experimental groups to which the liquid herbal compositions were administered showed less bad results. This is because the liquid herbal composition inhibited osteoclast activity, and thus it can be seen that the liquid herbal composition can be actively used for the treatment and prevention of osteoporosis.

## Claims

1. An herbal composition in powder, pill or liquid form for use in speeding up fracture healing and bone regeneration containing 20 parts by weight of Angelica gigas roots in dry state, 16 parts by weight of Dendrobium moniliforme stem in dry state, 12 parts by weight of Dipsaci radix roots in dry state, 10 parts by weight of ossified horns of antler in dry state, 8 parts by weight of Cnidium officinale roots in dry state, 8 parts by weight of Astragalus membranaceus roots in dry state, and 4 parts by weight of Achyranthes japonica roots in dry state.

2. An herbal composition in powder, pill or liquid form for use according to claim 1, further containing 2 parts by weight of Massa medicate fermentata fruit in dry state.

3. An herbal composition in powder, pill or liquid form for use in speeding up fracture healing and bone regeneration containing 10 parts by weight of Angelica gigas roots in dry state, 8 parts by weight of Dendrobium moniliforme stem in dry state, 6 parts by weight of Dipsaci radix roots in dry state, 5 parts by weight of ossified horns of antler in dry state, 4 parts by weight of Cnidium officinale roots in dry state, 4 parts by weight of Astragalus membranaceus roots in dry state, 2 parts by weight of Achyranthes japonica roots in dry state, and 4 parts by weight of Massa medicate fermentata fruit in dry state.

4. An herbal composition in powder, pill or liquid form for use according to claim 1, further containing 4 parts by weight of Massa medicate fermentata fruit in dry state.

5. An herbal formulation in powder, pill or liquid form for use in speeding up fracture healing and bone regeneration, which contains, as an active ingredient, the herbal composition of any one of claims 1 to 4, and is prepared in powder, pill or liquid form.

## Patentansprüche

1. Kräuterzusammensetzung in Pulver-, Pillen- oder Flüssigform zur Verwendung zur Beschleunigung der Frakturheilung und Knochenregeneration, aufweisend 20 Gewichtsteile Angelica gigas-Wurzeln im getrockneten Zustand, 16 Gewichtsteile Dendrobium moniliforme-Stängel im getrockneten Zustand, 12 Gewichtsteile Dipsaci radix-Wurzeln im getrockneten Zustand, 10 Gewichtsteile verknöcherte Geweihhörner im getrockneten Zustand, 8 Gewichtsteile Cnidium officinale-Wurzeln im getrockneten Zustand, 8 Gewichtsteile Astragalus membranaceus-Wurzeln im getrockneten Zustand und 4 Gewichtsteile Achyranthes japonica-Wurzeln im getrockneten Zustand.

2. Kräuterzusammensetzung in Pulver-, Pillen- oder Flüssigform zur Verwendung nach Anspruch 1, weiter aufweisend 2 Gewichtsteile Massa medicate fermentata-Früchte im getrockneten Zustand.

3. Kräuterzusammensetzung in Pulver-, Pillen- oder Flüssigform zur Verwendung zur Beschleunigung der Frakturheilung und Knochenregeneration, aufweisend 10 Gewichtsteile Angelica gigas-Wurzeln im getrockneten Zustand, 8 Gewichtsteile Dendrobium moniliforme-Stängel im getrockneten Zustand, 6 Gewichtsteile Dipsaci radix-Wurzeln im getrockneten Zustand, 5 Gewichtsteile verknöcherte Geweihhörner im getrockneten Zustand, 4 Gewichtsteile Cnidium officinale-Wurzeln im getrockneten Zustand, 4 Gewichtsteile Astragalus membranaceus-Wurzeln im getrockneten Zustand, 2 Gewichtsteile Achyranthes japonica-Wurzeln im getrockneten Zustand und 4 Gewichtsteile Massa medicate fermentata-Früchte im getrockneten Zustand.

4. Kräuterzusammensetzung in Pulver-, Pillen- oder Flüssigform zur Verwendung nach Anspruch 1, weiter aufweisend 4 Gewichtsteile Massa medicate fermentata-Früchte im getrockneten Zustand.

5. Kräuterformulierung in Pulver-, Pillen- oder Flüssigform zur Verwendung zur Beschleunigung der Frakturheilung und Knochenregeneration, die als einen Wirkstoff die Kräuterzusammensetzung nach einem der Ansprüche 1 bis 4 aufweist, und in Pulver-, Pillen- oder Flüssigform hergestellt ist.

## Revendications

1. Composition à base de plantes médicinales, sous forme de poudre, de pilule ou de liquide, destinée à être utilisée pour accélérer la consolidation des fractures et la régénération osseuse, contenant 20 parties en poids de racines d'*Angelica gigas* à l'état sec, 16 parties en poids de tiges de *Dendrobium moniliforme* à l'état sec, 12 parties en poids de racines de *Dipsacus* à l'état sec, 10 parties en poids de bois de cerf ossifiés à l'état sec, 8 parties en poids de racines de *Cnidium officinale* à l'état sec, 8 parties en poids de racines d'*Astragalus membranaceus* à l'état sec, et 4 parties en poids de racines d'*Achyranthes japonica* à l'état sec.

2. Composition à base de plantes médicinales, sous forme de poudre, de pilule ou de liquide, destinée à être utilisée selon la revendication 1, contenant en outre 2 parties en poids de fruit de Massa medicata fermentata à l'état sec.

3. Composition à base de plantes médicinales, sous forme de poudre, de pilule ou de liquide, destinée à être utilisée pour accélérer la consolidation des fractures et la régénération osseuse, contenant 10 parties en poids de racines d'*Angelica gigas* à l'état sec, 8 parties en poids de tiges de *Dendrobium moniliforme* à l'état sec, 6 parties en poids de racines de *Dipsacus* à l'état sec, 5 parties en poids de bois de cerf ossifiés à l'état sec, 4 parties en poids de racines de *Cnidium officinale* à l'état sec, 4 parties en poids de racines d'*Astragalus membranaceus* à l'état sec, 2 parties en poids de racines d'*Achyranthes japonica* à l'état sec, et 4 parties en poids de fruit de Massa medicata fermentata à l'état sec

4. Composition à base de plantes médicinales, sous forme de poudre, de pilule ou de liquide, destinée à être utilisée selon la revendication 1, contenant en outre 4 parties en poids de fruit de Massa medicata fermentata à l'état sec.

5. Formulation à base de plantes médicinales, sous forme de poudre, de pilule ou de liquide, destinée à accélérer la consolidation des fractures et la régénération osseuse, qui contient, en tant que composant actif, la composition à base de plantes médicinales selon l'une quelconque des revendications 1 à 4, et est préparée sous forme de poudre, de pilule ou de liquide.
